# EUROPÄISCHE PATENTSCHRIFT

(11) **EP 2 878 948 B1**
(45) Veröffentlichungstag und Bekanntmachung des Hinweises auf die Patenterteilung: **01.08.2018**
(21) Anmeldenummer: 13195328.3
(22) Anmeldetag: 02.12.2013
(51) Int. Cl.: G01N 33/18

(54) **Verfahren und Vorrichtung zur Wasserqualitätsüberwachung**
Method and device for water quality monitoring
Procédé et dispositif de surveillance de la qualité de l'eau

(43) Veröffentlichungstag der Anmeldung: 03.06.2015
(73) Patentinhaber: Airbus Defence and Space GmbH, 82024 Taufkirchen (DE)
(72) Erfinder: Kern, Peter, 88682 Salem (DE)
(74) Vertreter: Daub, Thomas

(56) Entgegenhaltungen:
- WO-A1-2007/086754
- FR-A1- 2 573 875
- US-A1- 2003 217 590

## Beschreibung

### Stand der Technik

Die Erfindung betrifft ein Verfahren zur Wasserqualitätsüberwachung mittels zumindest einer Überwachung von Eigenbewegungen lebender Testorganismen, insbesondere Fischen und/oder Daphnien, in einem Wasservolumen, nach dem Oberbegriff des Anspruchs 1 und eine entsprechende Vorrichtung.

Es sind bereits Verfahren zur Wasserqualitätsüberwachung mittels zumindest einer Überwachung von Eigenbewegungen lebender Testorganismen bekannt. So beispielsweise der im Jahr 1971 entwickelte Kerren-Strömungsfischtest, in dem Goldorfen gegen eine periodische erzeugte Strömung anschwimmen und bei einer durch toxische Stoffe im Wasser bewirkten Beeinträchtigung ihrer Schwimmfähigkeit abgetrieben werden, welcher heutzutage aus Gründen des Tierschutzes nicht mehr angewandt wird. Weiterhin in Gebrauch ist der 1978 entwickelte dynamische Daphnientest, in dem mittels Photozellen oder Videokameras die Eigenbewegungen von Krebstieren der Gattung Daphnia überwacht werden. Daphnien reagieren empfindlich auf Schadstoffe im Wasser und sterben daher bei Schadstoffbelastung des Wassers rasch ab. Daphnien bewegen sich sehr rasch und sind in einem Normalzustand beinahe stets in Bewegung, so dass eine Einstellung der Eigenbewegung als Beweis für ein Absterben der Daphnien angesehen werden kann. Durch eine Überwachung der Bewegung der Daphnien mittels der Photozellen oder Videokameras ist eine Überwachung der Wasserqualität möglich, da ein Einstellen einer Eigenbewegung durch einen großen Teil der Daphnien oder durch alle Daphnien auf eine Schadstoffbelastung hinweist. Eine sichere optische Überwachung von Eigenbewegungen lebender Testorganismen erfordert einen hohen apparativen und rechnerischen Aufwand. FR2573875 A1 offenbart ein Verfahren zur Wasserqualitätsüberwachung mittels einer Überwachung von Eigenbewegungen lebender Fische, in einem Wasservolumen, wobei die Eigenbewegungen der lebenden Fische mittels Schalldopplermessung überwacht werden.

US2003217590 A1 offenbart ein Verfahren zur Wasserqualitätsüberwachung mittels zumindest einer Überwachung von Eigenbewegungen lebender Fische, in einem Wasservolumen, wobei die Eigenbewegungen der lebenden Fische mittels Schallmessung überwacht werden, und wobei über einen Meßzeitraum gemessene Schallsignale miteinander verglichen werden, wobei aus einer Veränderung der Signale über den Meßzeitraum eine Verhaltensänderung der lebenden Fische während eines Verlaufs des Meßzeitraums bestimmt wird. US2003217590 A1 offenbart zudem, dass zur Überwachung auch die Bewegungsfrequenz und die zugehörige Amplitude, d.h. Größen die mit der Geschwindigkeit der Fische zusammenhängen, herangezogen werden. Die Aufgabe der Erfindung besteht insbesondere darin, ein gattungsgemäßes Verfahren mit einem verringerten Aufwand bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können. Die Erfindung betrifft zudem eine Vorrichtung gemäß Anspruch 10.

### Vorteile der Erfindung

Die Erfindung geht aus von einem Verfahren zur Wasserqualitätsüberwachung mittels zumindest einer Messung von Eigenbewegungen lebender Testorganismen, insbesondere Fischen und/oder Daphnien, in einem Wasservolumen.

Es wird vorgeschlagen, dass die Eigenbewegungen der lebenden Testorganismen mittels Schalldopplermessung überwacht werden. Unter "Eigenbewegungen" sollen Bewegungen der lebenden Testorganismen verstanden werden, die sie durch eigene Kraft durchführen, insbesondere Schwimmbewegungen und Körperteilbewegungen wie beispielsweise Kiemenbewegungen. Eigenbewegungen sind verschieden von passiven Bewegungen, wie beispielsweise einem Abtreiben unter einer Strömungseinwirkung. Unter einer "Wasserqualität" soll insbesondere eine Schadstoffbelastung eines stehenden oder fließenden Gewässers oder eines Inhalts einer Wasserleitung, beispielsweise Frischwasser, Abwasser oder Brauchwasser, verstanden werden, wobei Schadstoffe von natürlichen oder künstlichen Toxinen, Medikamentenrückständen, gelösten flüchtigen organischen Verbindungen, Düngemittelrückständen, organischen Lösungsmitteln, und/oder von Tieren oder Pflanzen, wie beispielsweise Algen, freigesetzte Stoffe gebildet sein können. Unter einer "Schallmessung" soll insbesondere eine Messung von Schallsignalen, die durch Eigenbewegungen der lebenden Testorganismen bewirkt werden, verstanden werden, wobei die Schallsignale grundsätzlich sowohl von bei der Bewegung direkt erzeugten Schallsignalen als auch bevorzugt von reflektierten Schallsignalen von gezielt eingebrachten Anregungssignalen gebildet sein kann. Im Wasser lebende Tiere, die als lebende Testorganismen zur Bestimmung einer Wasserqualität eingesetzt werden, erzeugen aufgrund von Schwimmbewegungen und Kiemenflossenbewegungen für eine Atmung charakteristische Muster von Schallsignalen, welche bei der Schallmessung registriert und erkannt werden können. Die lebenden Testorganismen reagieren zudem auf eine Schadstoffbelastung mit einer gesteigerten Aktivität und Hektik. Die charakteristischen Muster von Schallsignalen unterscheiden sich stark voneinander, je nachdem, ob ein normales Schwimm- und Atemverhalten oder ein Verhalten mit gesteigerter Aktivität und Hektik aufgrund der Schadstoffbelastung vorliegt, so dass anhand gemessener charakteristischer Muster eine Bestimmung einer Schadstoffbelastung möglich ist. Eine Schadstoffbelastung, die eine Lähmung oder ein Sterben der lebenden Testorganismen bewirkt, kann über eine Reduktion oder ein weitgehendes Ausbleiben aller für eine Bewegung charakteristischen Schallsignale erkannt werden. Insbesondere tritt das Verhalten mit gesteigerter Aktivität und Hektik aufgrund der Schadstoffbelastung bei den lebenden Testorganismen zu einem Zeitpunkt nach Beginn der Schadstoffbelastung auf, bei denen noch keine Beeinträchtigung einer Schwimm- und/oder Lebensfähigkeit eintritt. Es kann insbesondere ein rasch durchzuführendes Verfahren mit einem geringen apparativen Aufwand zur Verfügung gestellt werden, mit dem bevorzugt zusätzlich eine Gefährdung der lebenden Testorganismen durch die Schadstoffbelastung verringert werden kann, indem bei einer Feststellung eines hektischen Verhaltens aufgrund der Schadstoffbelastung die lebenden Testorganismen aus schadstoffbelastetem Wasser entfernt werden.

Die Schallmessung kann in einem kontinuierlichen Betrieb oder in einem diskontinuierlichen Betrieb erfolgen. Unter einem "kontinuierlichen Betrieb" der Schallmessung soll insbesondere ein Betrieb verstanden werden, bei dem eine Schallsensoreinheit und eine separat von der Schallsensoreinheit ausgeführte Schallerregereinheit permanent aktiviert sind oder ein Betrieb, in dem eine einzelne Einheit zwischen Schallaussendung und Schallempfang umgeschaltet wird, wobei eine Empfangsphase unmittelbar auf eine Aussendungsphase folgt und umgekehrt. Ein kontinuierlicher Betrieb wird auch als "continuous wave"-Betrieb oder cw-Betrieb bezeichnet. Unter einem "diskontinuierlichen Betrieb" der Schallmessung soll insbesondere ein Betrieb verstanden werden, bei denen zwischen Aussendungsphasen und/oder Empfangsphasen Pausen liegen, in denen weder eine Aussendung von Anregungssignalen noch ein Empfang von Schallsignalen erfolgt.

Des Weiteren wird vorgeschlagen, dass die Eigenbewegungen der lebenden Testorganismen mittels Schalldopplermessung überwacht werden. Unter einer "Schalldopplermessung" soll insbesondere eine Messung verstanden werden, bei der Anregungssignale in das Wasservolumen eingestrahlt und an belebten und/oder unbelebten Objekten in dem Wasservolumen oder an Grenzflächen das Wasservolumens reflektiert werden, wobei sich aufgrund einer Bewegung der belebten und/oder unbelebten Objekte relativ zu einer Quelle der Anregungssignale während einer Signaldauer eine Frequenzverschiebung bei Schallsignalen, die von reflektierten Anregungssignalen gebildet sind, ergibt. Ein Auftreten der Frequenzverschiebung wird als Dopplereffekt bezeichnet. Insbesondere ist das Verhalten mit gesteigerter Aktivität und Hektik der lebenden Testorganismen in einem Spektrum gemessener Schallsignale durch eine Verschiebung von Dopplerfrequenzen der Schallsignale zu höheren Frequenzen und durch eine größere Signalintensität als bei einem Verhalten unter Normalbedingungen gekennzeichnet. Entsprechend kann eine Schadstoffbelastung, die eine Lähmung oder ein Absterben der lebenden Testorganismen bewirkt hat, dadurch detektiert werden, dass dopplerverschobene Signale ausbleiben. Es kann insbesondere eine Schallmessung unter weitgehend kontrollierten Messbedingungen und eine große Erfassungswahrscheinlichkeit der lebenden Testorganismen erreicht werden.

Ferner wird vorgeschlagen, dass die Eigenbewegungen der lebenden Testorganismen mittels Ultraschallmessung überwacht werden. Unter einer "Ultraschallmessung" soll eine Schallmessung von als Ultraschallsignalen ausgebildeten Schallsignalen, die eine Frequenz im als Ultraschall bezeichneten Frequenzbereich aufweisen, verstanden werden. Unter einem "Ultraschallsignal" soll insbesondere ein Schallsignal in einem Frequenzbereich oberhalb der menschlichen Hörschwelle verstanden werden, insbesondere in einem Frequenzbereich zwischen 16 kHz bis 1 GHz, bevorzugt zwischen 500 kHz bis 20 MHz. Grundsätzlich können in einer alternativen Ausgestaltung auch alternativ Infraschallsignale mit Frequenzen unterhalb der menschlichen Hörschwelle verwendet werden. Insbesondere sind die lebenden Testorganismen dahingehend ausgewählt, dass Ultraschall von den lebenden Testorganismen nicht detektiert wird. Es kann insbesondere ein Verfahren zur Wasserqualitätsüberwachung erhalten werden, das eine Schallbelastung von Menschen und/oder den lebenden Testorganismen vermeidet.

In einer Weiterbildung der Erfindung wird vorgeschlagen, dass gemessene Schallsignale mit zumindest einem Referenzsignal für eine Eigenbewegung der lebenden Testorganismen bei Normalbedingungen verglichen werden. Insbesondere werden die gemessenen Schallsignale zuvor in eine bestimmte Signalform umgewandelt, beispielsweise in ein Frequenzspektrum oder ein Amplitudensignal. Unter einem "Frequenzspektrum" soll insbesondere ein Spektrum von gemessenen Schallsignalen verstanden werden, bei dem über einen bestimmten Zeitraum gemessene Schallsignale nach ihrer Frequenz aufgelöst sind. Unter einem "Amplitudensignal" soll insbesondere ein Signal verstanden werden, bei dem über einen definierten Zeitraum Spannungssignale, die mittels Frequenz-Spannungs-Wandlung aus den gemessenen Schallsignalen gewonnen werden, verwendet werden. Erfindungsgemäß werden die gemessenen Schallsignale als Dopplersignale ausgewertet. Insbesondere wird das Dopplersignalspektrum dadurch bestimmt, dass eine Differenzfrequenz von Frequenzen gemessener Schallsignale zu einer Frequenz von Anregungssignalen bestimmt wird. Unter einem "Referenzsignal" soll insbesondere ein in der Speichereinheit hinterlegtes Signal, beispielsweise ein Schwellenwert für ein Amplitudensignal oder ein Referenzspektrum, verstanden werden, das ein Verhalten der lebenden Testorganismen bei einer definierten Schadstoffbelastung charakterisiert. Insbesondere entspricht das Referenzsignal einem Verhalten der lebenden Testorganismen bei einer schädlichen Schadstoffbelastung des Wassers. Unter "Normalbedingungen" sollen Bedingungen mit einer geringen, für die lebenden Testorganismen unschädlichen Schadstoffbelastung des Wassers verstanden werden. Insbesondere werden die Signalspektren gemessener Schallsignale in zumindest einer Auswerteeinheit mit zumindest einem Referenzspektrum verglichen. Unter einer "Auswerteeinheit" soll insbesondere eine Einheit mit zumindest einer Datenempfangseinheit, mit einer Prozessoreinheit und mit einer Speichereinheit sowie mit einem in der Speichereinheit gespeicherten Betriebsprogramm verstanden werden, die zu einer Auswertung von empfangenen Daten vorgesehen ist. Insbesondere wird das Signalspektrum von der Auswerteeinheit aus Messwerten einer Schallsensoreinheit erstellt. Insbesondere kann das Signalspektrum in Echtzeit oder durch Integration von über einen definierten Zeitraum gemessenen Schallsignalen erstellt werden. Insbesondere können mehrere Referenzsignale verwendet werden, beispielsweise ein Referenzsignal für ein hektisches Verhalten und ein Referenzsignal für Eigenbewegungen der lebenden Testorganismen, welche aufgrund von Schadstoffbelastung gelähmt sind. Auch können mehrere Referenzsignale für Normalverhalten zu unterschiedlichen Zeitpunkten verwendet werden, um beispielsweise nachlassende Aktivität der lebenden Testorganismen während einer Nachtzeit zu berücksichtigen und eine Fehlinterpretation als Eigenbewegungen bei Lähmung zu vermeiden. Es kann insbesondere eine einfach und automatisiert durchführbare Bestimmung der Wasserqualität erreicht werden.

Ferner wird vorgeschlagen, dass über einen Meßzeitraum gemessene Signale miteinander verglichen werden. Erfindungsgemäß wird aus einer Veränderung der Signale über den Meßzeitraum eine Verhaltensänderung der lebenden Testorganismen während eines Verlaufs des Meßzeitraums und eine Zunahme oder Abnahme einer Hektik aufgrund einer im Verlauf des Meßzeitraums zunehmenden oder abnehmenden Frequenzverschiebung der Schallsignale bestimmt. Der Meßzeitraum kann insbesondere eine feste, voreingestellte Zeitdauer umfassen, während der die Signale miteinander verglichen werden und nach deren Ablauf ein Auswerteergebnis ausgegeben wird, oder der Meßzeitraum kann von einem manuell oder fernbedient eingestellten Startzeitpunkt an ablaufen, bis er durch ein manuell oder fernbedient eingestelltes Endsignal abgebrochen wird, wobei während des Meßzeitraums ein Warnsignal ausgesandt werden kann, wenn die Auswerteeinheit eine Überschreitung eines Signalgrenzwerts feststellt, die mit einer Überschreitung einer Schadstoffbelastungsschwelle korrespondiert, oder wenn charakteristische Dopplersignale lebender Testorganismen ausbleiben, was mit deren Lähmung oder Absterben korrespondiert und/oder nach Beendigung des Meßzeitraums ein Auswerteergebnis ausgegeben werden kann. Es kann insbesondere ein Verfahren zur Verfügung gestellt werden, das bei einer Vielzahl unterschiedlicher lebender Testorganismen, insbesondere bei Testorganismen, für welche noch kein Referenzsignal in der Speichereinheit hinterlegt ist, verwendet werden kann. Insbesondere werden über den Meßzeitraum gemessene Dopplersignalspektren, insbesondere Ultraschalldopplersignalspektren, miteinander verglichen. Es kann insbesondere eine einfach und automatisiert durchführbare Bestimmung der Wasserqualität erreicht werden.

Des Weiteren wird vorgeschlagen, dass gemessene Schallsignale mittels eines numerischen Verfahrens ausgewertet werden. Unter einem "numerischen Verfahren" soll insbesondere ein Verfahren verstanden werden, bei dem eine approximative Auswertung von Messdaten durchgeführt wird. Beispielsweise kann das numerische Verfahren von einer Fast-Fourier-Transform, einer Wavelet-Analyse oder einem weiteren, einem Fachmann als geeignet erscheinenden numerischen Verfahren gebildet sein. Insbesondere kann mittels des numerischen Verfahrens eine kontinuierliche Überwachung der Wasserqualität durch eine regelmäßige oder kontinuierliche Erstellung von Signalspektren aus gemessenen Schallsignalen erreicht werden. Es kann insbesondere ein mit einem geringen zeitlichen Aufwand durchzuführendes Auswerteverfahren erreicht werden.

Ferner wird vorgeschlagen, dass gemessene Schallsignale mittels eines Filterverfahrens ausgewertet werden. Unter einem "Filterverfahren" soll insbesondere ein Verfahren verstanden werden, bei dem mittels eines analogen oder digitalen Filters einzelne Frequenzbereiche, Gradienten und/oder Schwellenwerte einer Signalintensität aus dem Signalspektrum betrachtet werden und bei Überschreitung eines bestimmten, charakteristischen Schwellenwerts, insbesondere eines für einen Zustand, der vor dem Zustand mit gesteigerter Hektik auftritt ein Signal ausgegeben wird. Insbesondere kann das Filterverfahren mittels analoger Filterelemente, die in der Schallsensoreinheit, einer Auswerteeinheit oder in einer Datenverbindung angeordnet sind, oder durch eine digitale Datenverarbeitung der Schallsignale durchgeführt werden. Es kann insbesondere ein mit einem geringen apparativen Aufwand durchführbares Verfahren erreicht werden.

Des Weiteren wird vorgeschlagen, dass ein Normalverhaltenssignal der lebenden Testorganismen dynamisch aus gemessenen Schallsignalen bestimmt wird. Unter "dynamisch aus gemessenen Schallsignalen bestimmt" soll insbesondere verstanden werden, dass die gemessenen Schallsignale während eines längeren, vordefinierten Zeitraums ausgewertet und ein aus den gemessenen Schallsignalen bestimmtes Signal, insbesondere eine Mittelwertsignal über den Zeitraum, als Normalverhaltenssignal genommen wird. Insbesondere wird die dynamische Bestimmung des Normalverhaltenssignals zu einem Beginn der Überwachung durchgeführt. Auch kann das Normalverhaltenssignal unter kontrollierten Bedingungen, bei bekannter sauberer Wasserqualität, aufgenommen werden. Das dynamisch bestimmte Normalverhaltenssignal kann einmalig bestimmt werden oder es kann in einem Beobachtungszeitraum ein adaptives, zeitlich veränderliches Normalverhaltenssignal bestimmt werden, indem gemessene Schallsignale einer Mittelwertbildung mit einer hohen Zeitkonstanten unterzogen werden und ein daraus bestimmter Mittelwert als aktuelles Normalverhaltenssignal verwendet wird. Insbesondere können die gemessenen Signale zur dynamischen Bestimmung des Normalverhaltenssignals mittels eines Filterverfahrens, beispielsweise eines Tiefpassfilterverfahrens, und/oder eines numerischen Verfahrens ausgewertet werden. Beispielsweise werden in einem vordefinierten Zeitraum gemessene Schallsignale spannungsgewandelt und mittels eines Tiefpassfilterverfahrens ausgewertet, so dass hochfrequente Schallsignale, die kurzfristigen, rapiden Eigenbewegungen der lebenden Testorganismen entsprechen, während des vordefinierten Zeitraums bei der Auswertung abgeschwächt werden und hauptsächlich niederfrequente Schallsignale, welche ruhigen Eigenbewegungen der lebenden Testorganismen entsprechen, im Wesentlichen unabgeschwächt durchgelassen. Die Spannungsignale nach Durchführung des Filterverfahrens werden anschließend über den Zeitraum aufintegriert und ein Mittelwert hieraus gebildet, welcher als Normalverhaltenssignal genutzt wird. Insbesondere kann eine dynamische Bestimmung des Normalverhaltenssignals über einen gesamten Einsatzzeitraums des Verfahrens permanent durchgeführt werden, wobei zusätzlich eine Überwachung von gemessenen Schallsignalen in kürzeren Zeiträumen innerhalb des Zeitraums, über den das Normalverhaltenssignal bestimmt wird, erfolgt, um aus kurzfristigen Verhaltensänderungen eine auftretende Schadstoffbelastung zu erkennen, oder in regelmäßigen oder unregelmäßigen Abständen wiederholt werden. In der Auswerteeinheit sind in der Speichereinheit Abweichungen von dem Normalverhaltenssignal nach oben und/oder nach unten festgelegt, welche einen Alarm auslösen, wobei eine Größe der Abweichungen nach oben verschieden sein kann von einer Größe der Abweichungen nach unten. Es kann insbesondere eine Anzahl an Fehlalarmen aufgrund einem sich im Lauf der Zeit änderndem Verhalten der lebenden Testorganismen, beispielsweise auftretende Schlafphasen, verringert werden.

Weiterhin wird vorgeschlagen, dass Anregungssignale zu einer Erzeugung von Schalldopplersignalen in einem kontinuierlichen Betrieb eingestrahlt werden. Unter einer "Einstrahlung von Anregungssignalen in einem kontinuierlichen Betrieb" soll insbesondere verstanden werden, das die Schallerregereinheit und eine von der Schallerregereinheit getrennt ausgebildete Schallsensoreinheit in einem als "continuous wave"-Betrieb oder cw-Betrieb bezeichneten Betrieb während eines Beobachtungszeitraums permanent aktiviert sind oder eine sowohl für eine Schallaussendung als auch Schallempfang ausgebildete Einheit in dem Beobachtungszeitraum in stetem Wechsel zwischen Schallaussendung und Schallempfang umgeschaltet wird. Es kann insbesondere eine konstante Überwachung der Wasserqualität erreicht werden.

Ferner wird vorgeschlagen, dass Anregungssignale zu einer Erzeugung von Schalldopplersignalen in einem diskontinuierlichen Betrieb eingestrahlt werden. Unter einem "diskontinuierlichen Betrieb" soll insbesondere ein Betrieb verstanden werden, bei dem zwischen Aussendungsphasen von Anregungssignalen und/oder Empfangsphasen von Schallsignalen Pausen liegen, in denen weder eine Aussendung von Anregungssignalen noch ein Empfang von Schallsignalen erfolgt. Es kann insbesondere ein Verfahren mit einem geringen energetischen Aufwand und mit einem geringen Rechenaufwand erreicht werden.

Des Weiteren wird eine Vorrichtung zur Durchführung des erfindungsgemäßen Verfahrens mit zumindest einem Testbehältnis für lebende Testorganismen, zumindest einer Schallsensoreinheit und zumindest einer Auswerteeinheit vorgeschlagen. Unter einem "Testbehältnis für lebende Testorganismen" soll ein Behältnis verstanden werden, das lebende Testorganismen in einem mit Wasser gefüllten Hohlraum einschließt. Insbesondere weist das Testbehältnis Wandelemente auf, die den Hohlraum umschließen, wobei in den Wandelementen verschließbare Öffnungen und/oder Perforationen vorhanden sein können. Insbesondere kann ein Wandelement als ein Gitterwandelement mit Perforationen ausgebildet sein, wobei eine Größe der Perforationen auf einen jeweiligen eingeschlossenen lebenden Testorganismus abgestimmt ist, um den lebenden Testorganismus an einem Passieren des Gitterwandelements zu hindern. Insbesondere sind verschließbare Öffnungen und/oder Perforationen dazu vorgesehen, einen Eintritt von Wasser, dessen Qualität bestimmt werden soll, in den Hohlraum des Testbehältnisses zu ermöglichen. Insbesondere kann das Testbehältnis als Gitterkäfig ausgebildet sein, der zu einer Durchführung einer Bestimmung der Wasserqualität in Wasser, dessen Qualität bestimmt werden soll, eingesetzt wird und vollständig von diesem durchströmt wird und der zu einer Bestimmung der Qualität in das Wasser eingeführt und anschließend mit lebenden Testorganismen besetzt wird. Insbesondere kann das Testbehältnis einen von Wandelementen freien Bereich aufweisen, der dazu vorgesehen ist, in einem Betrieb der Vorrichtung mit einer Wasseroberfläche abzuschließen oder aus dieser hervorzustehen. Insbesondere ist der von Wandelementen freie Bereich dazu vorgesehen, ein Hinzusetzen oder eine Entnehmen von Testorganismen und/oder von Funktionseinheiten wie der zumindest einen Schallsensoreinheit zu ermöglichen. Bevorzugt weist das Testbehältnis eine quaderförmige, zylindrische oder kreisscheibenförmige Form auf, die an maximal einer Seite offen ist. Insbesondere kann das Testbehältnis eine verschließbare Öffnung, beispielsweise eine durch eine Klappe verschließbare Öffnung, aufweisen. Erfindungsgemäß umfasst die Vorrichtung ferner eine Schallerregereinheit zu einer Aussendung von Anregungssignalen. Unter einer "Schallerregereinheit" soll eine Einheit verstanden werden, welche Schallsignale erzeugt und gerichtet oder ungerichtet abstrahlt. Unter einer "Schallsensoreinheit" soll eine Einheit mit zumindest einem Schallsensorelement zu einer Detektion von Schallsignalen verstanden werden. Insbesondere ist das Schallsensorelement als Signalwandler ausgebildet, der von dem Schallsensorelement detektierte Schallsignale in elektrische Signale umwandelt, beispielsweise als ein elektroakustischer Wandler, ein magnetostriktiver Wandler oder als ein Signalwandler, der zumindest ein Piezo-Element aufweist. Es kann insbesondere eine kompakte, universell einsetzbare Vorrichtung zur Durchführung des erfindungsgemäßen Verfahrens zur Verfügung gestellt werden.

In einer Weiterbildung der Erfindung wird vorgeschlagen, dass das zumindest eine Testbehältnis für die lebenden Testorganismen von einem verschließbaren Wassertank gebildet ist. Es kann insbesondere ein sicherer Einschluss der lebenden Testorganismen erreicht werden.

Weiterhin wird vorgeschlagen, dass der verschließbare Wassertank zumindest eine Schrägwand zu einer Umlenkung von Schallsignalen aufweist. Unter einer "Schrägwand" soll ein Wandelement des Wassertanks verstanden werden, das in einem von einem rechten Winkel verschiedenen Winkel zu zumindest einem weiteren Wandelement des Wassertanks angeordnet ist. Insbesondere werden durch die zumindest eine Schrägwand Schallsignale reflektiert, sodass ein Volumen des verschließbaren Wassertanks mit einer hohen Sicherheit vollständig durch die Schallsignale erfasst wird. Es kann insbesondere eine hohe Erfassungsrate der lebenden Testorganismen und somit eine Sicherheit bei einer Bestimmung der Wasserqualität erreicht werden.

Des Weiteren wird vorgeschlagen, dass das zumindest eine Testbehältnis für die lebenden Testorganismen von einem Gitterkäfig gebildet ist. Unter einem "Gitterkäfig" soll insbesondere ein Behältnis mit aus Gitterstangen bestehenden Wandelementen verstanden werden. Ein Abstand der Gitterstangen untereinander ist derart gewählt, dass die lebenden Testorganismen in einem Innenvolumen des Gitterkäfigs verbleiben und nicht aus diesem herauskommen können. Insbesondere kann das zumindest eine Testbehältnis mindestens eine öffnenbare Klappe aufweisen, durch die die lebenden Testorganismen in das Innenvolumen eingesetzt werden können oder aus diesem entfernt werden können. Insbesondere kann ein Wandelement vollständig von den anderen Wandelementen abnehmbar oder von den anderen Wandelementen wegklappbar ausgebildet sein. Insbesondere wird das zumindest eine Testbehältnis in ein zu testendes Wasservolumen eingeführt und anschließend die lebenden Testorganismen durch die öffnenbare Klappe eingesetzt. Es kann insbesondere eine Vorrichtung mit einem einfach herstellbaren und ein geringes Gewicht aufweisenden Testbehältnis erreicht werden.

Ferner wird vorgeschlagen, dass die zumindest eine Schallsensoreinheit zumindest ein erstes Schallsensorelement zu einer Aufnahme von Schallsignalen entlang einer ersten Raumrichtung des zumindest einen Testbehältnisses und zumindest ein zweites Schallsensorelement zu einer Aufnahme von Schallsignalen entlang einer zweiten Raumrichtung des zumindest einen Testbehältnisses aufweist. Es kann insbesondere eine hohe Auflösungsqualität der Schallmessung erreicht werden.

Die erfindungsgemäße Vorrichtung zur Durchführung des Verfahrens soll hierbei nicht auf die oben beschriebene Anwendung und Ausführungsform beschränkt sein. Insbesondere kann die erfindungsgemäße Vorrichtung zu einer Erfüllung einer hierin beschriebenen Funktionsweise eine von einer hierin genannten Anzahl von einzelnen Elementen, Bauteilen und Einheiten abweichende Anzahl aufweisen, solange sie unter den Schutzumfang des Anspruchs 10 fällt.

### Zeichnungen

Weitere Vorteile ergeben sich aus der folgenden Zeichnungsbeschreibung. In den Zeichnungen sind sieben Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer Vorrichtung zur Durchführung eines Verfahrens zur Wasserqualitätsüberwachung mittels einer Überwachung von Eigenbewegungen lebender Testorganismen in einem Wasservolumen, wobei die Eigenbewegungen der lebenden Testorganismen mittels Schallmessung überwacht werden, mit einem Testbehältnis für die lebenden Testorganismen, das als verschließbarer Wassertank ausgebildet ist,
- Fig. 2: eine Darstellung der Vorrichtung schräg von einer Seite,
- Fig. 3: eine Darstellung beispielhafter Amplitudensignale von gemessenen Schallsignalen, wie sie in einem normalen Zustand der lebenden Testorganismen, welche sporadische, kurzfristige Aktivitätsphasen zeigen, auftreten,
- Fig. 4: eine Darstellung beispielhafter Amplitudensignale von gemessenen Schallsignalen, wie sie in einem überwiegend hektischen Zustand der lebenden Testorganismen mit einem gegenüber dem normalen Zustand deutlich erhöhter Aktivität auftreten,
- Fig. 5: eine Darstellung einer alternativen Vorrichtung, bei der eine Schallsensoreinheit ein erstes Schallsensorelement und ein zweites Schallsensorelement aufweist, welche unterschiedliche Raumrichtungen erfassen, schräg von einer Seite,
- Fig. 6: eine Darstellung einer zweiten alternativen Vorrichtung, in dem ein verschließbarer Wassertank eine Schrägwand aufweist,
- Fig. 7: eine Darstellung einer dritten alternativen Vorrichtung, in dem ein verschließbarer Wassertank eine doppelt geneigte Schrägwand aufweist,
- Fig. 8: eine Darstellung einer vierten alternativen Vorrichtung mit einer Zylinderform,
- Fig. 9: eine Darstellung einer fünften alternativen Vorrichtung mit einem Testbehältnis, das als eintauchbarer Gitterkäfig ausgebildet ist und
- Fig. 10: eine Darstellung einer sechsten alternativen Vorrichtung, in dem ein verschließbarer Wassertank eine zweifach gekippte Schrägwand aufweist.

### Beschreibung der Ausführungsbeispiele

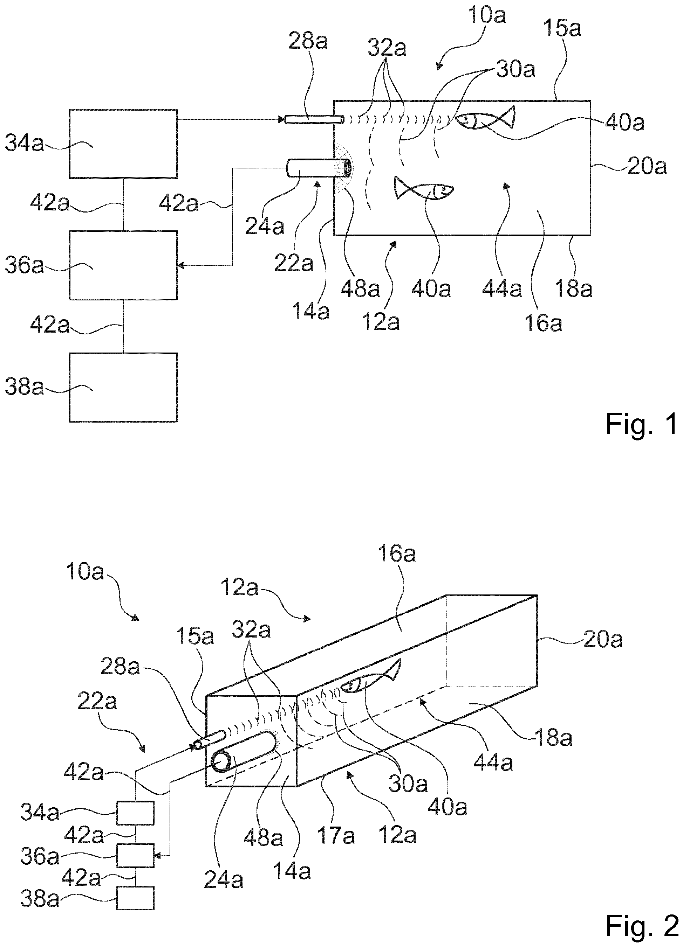

Fig. 1 zeigt eine schematische Darstellung einer Vorrichtung 10a zur Durchführung eines Verfahrens zur Wasserqualitätsüberwachung mittels einer Überwachung von Eigenbewegungen lebender Testorganismen 40a in einem Wasservolumen 44a, wobei die Eigenbewegungen der lebenden Testorganismen 40a mittels Schallmessung überwacht werden. Als lebende Testorganismen 40a werden Fische eingesetzt. Auch können anstelle von Fischen andere Wasserlebewesen oder aquatische Lebewesen wie beispielsweise Daphnien, Ruderfußkrebse oder Molche eingesetzt werden. Die Vorrichtung 10a umfasst ein Testbehältnis 12a für die lebenden Testorganismen 40a mit sechs Wandelementen 14a, 15a, 16a, 17a, 18a, 20a (Fig. 2), eine Schallsensoreinheit 22a mit einem ersten Schallsensorelement 24a, das einen Piezo-Wandler aufweist, eine Signalgeneratoreinheit 34a, die eine Schallerregereinheit 28a antreibt, eine Signalwandlungseinheit 36a zur Umwandlung empfangener Schallsignale und zur Bestimmung einer Frequenzverschiebung und eine Auswerteeinheit 38a, die durch die Schallsensoreinheit 22a gemessene und in der Signalwandlungseinheit 36a umgewandelte Schallsignale 30a auswertet. Die Auswerteeinheit 38a ist über von Kabeln gebildete Datenübertragungselemente 42a mit der Signalwandlungseinheit 36averbunden. Das Testbehältnis 12a für lebende Testorganismen 40a ist von einem verschließbaren Wassertank gebildet, der massive Wandelemente 14a, 16a, 18a, 20a aus Glas aufweist, welche einen Hohlraum, in welchen das Wasservolumen 44a mit den darein gesetzten lebenden Testorganismen 40a aufgenommen ist, begrenzen. Anstelle aus Glas können die Wandelemente 14a, 16a, 18a, 20a auch aus Metall oder Kunststoff bestehen. Der Wassertank weist eine nicht dargestellte, verschließbare Klappe auf, durch die Wasser, welches auf eine Schadstoffbelastung überprüft werden soll, in den Hohlraum eingebracht wird und die lebenden Testorganismen 40a in das Wasservolumen 44a gesetzt werden. Das Testbehältnis 12a ist dazu vorgesehen, in eine größere Wassermenge eingesetzt und durch Öffnung der Klappe mit dem Wasservolumen 44a gefüllt zu werden. Alternativ kann das Testbehältnis 12a an Land aufgestellt und anschließend mit Wasser befüllt werden. Das erste Schallsensorelement 24a ist mit einem Schutzelement 48a ausgestattet, welches Beschädigungen und Fehlsignale des ersten Schallsensorelements 24a, die durch Verbiss des ersten Schallsensorelements 24a durch die lebenden Testorganismen 40a auftreten können, verhindert. Das Schutzelement 48a ist als Gitterelement mit einer Vielzahl von Öffnungen ausgebildet und umgibt das erste Schallsensorelement 24a vollständig.

Die Eigenbewegungen der lebenden Testorganismen 40a werden mittels Schalldopplermessung überwacht, bei der Schallsignale 30a gemessen werden, welche von Reflektionen von Anregungssignalen 32a, die von der Schallerregereinheit 28a ausgesandt werden, gebildet sind. Die Schallerregereinheit 28a wird von der Signalgeneratoreinheit 34a angetrieben, welche einen Tonfrequenzgenerator zur Erzeugung der Steuersignale für die Schallerregereinheit 28a aufweist. Reflektierte Anregungssignale 32a, die von dem ersten Schallsensorelement 24a aufgenommen werden, bilden die auszuwertenden Schallsignale 30a. Die Anregungssignale 32a werden an den lebenden Testorganismen 40a reflektiert, wobei sich aufgrund einer relativen Bewegung der lebenden Testorganismen 40a zu der Schallerregereinheit 28a eine Dopplerverschiebung der Schallsignale 30a gegenüber den Anregungssignalen 32a ergibt. Durch Messung der Schallsignale 30a können somit die Eigenbewegungen der lebenden Testorganismen 40a überwacht werden. Die lebenden Testorganismen 40a reagieren auf eine Schadstoffbelastung des Wassers mit einer Verhaltensänderung gegenüber einem Verhalten unter Normalbedingungen, bei welcher höchstens eine geringe Schadstoffbelastung des Wassers vorhanden ist. Auf eine Schadstoffbelastung des Wassers reagieren die lebenden Testorganismen 40a mit einer gesteigerten Unruhe und schnelleren Eigenbewegungen, die in Abhängigkeit von einer Stärke der Schadstoffbelastung bis zu Fluchtbewegungen reichen. Die schnelleren Eigenbewegungen können in dem Dopplerspektrum anhand einer Position und Amplitudenhöhe von Amplitudenpeaks bestimmt werden. Die Position ist durch eine Zentralfrequenz des Amplitudenpeaks gegeben. Eine Position von Amplitudenpeaks, die bei dem Verhalten mit gesteigerter Unruhe auftauchen, kann beispielsweise durch Vergleichsmessungen bei bekannten Schadstoffkonzentrationen vorher ermittelt worden sein. Alternativ kann ein Vergleichsspektrum der lebenden Testorganismen 40a in einem Normalzustand in unbelastetem Wasser aufgenommen werden und das Verhalten mit gesteigerter Unruhe - und somit indirekt die Schadstoffbelastung - über eine Überschreitung eines vordefinierten Grenzwerts einer Verschiebung der Amplitudenpeaks gegenüber Amplitudenpeaks des Vergleichsspektrum detektiert werden. Die gesteigerte Unruhe macht sich nicht nur in schnelleren Schwimmbewegungen der lebenden Testorganismen 40a bemerkbar, sondern auch in einer erhöhten Atemfrequenz der lebenden Testorganismen 40a. Eine erhöhte Atemfrequenz der lebenden Testorganismen 40a ist durch eine Erhöhung einer Frequenz von Bewegungen von Kiemenflossen gekennzeichnet. Eine erhöhte Atemfrequenz wird erfindungsgemäß somit durch eine Intensität und Frequenz eine Kiemenflossenbewegungen zugeordneten Amplitudenspitze in einem als Dopplerspektrum ausgebildeten Signalspektrum von Schallsignalen 30a detektiert. Die lebenden Testorganismen 40a zeigen ein geändertes Verhalten schon bei Schadstoffkonzentrationen, welche für sie unschädlich sind. Bei einer sehr hohen Schadstoffkonzentration kann es zu einer Schädigung der lebenden Testorganismen 40a kommen, welche zu einer teilweisen oder vollständigen Lähmung oder zum Tod der Testorganismen 40a führt. Eine solche Schadstoffkonzentration kann in dem Dopplerspektrum dadurch detektiert werden, dass Amplitudenpeaks, die den Eigenbewegungen der Testorganismen 40a entsprechen, zu niedrigeren Frequenzen gegenüber dem Normalzustand verschoben sind oder vollständig wegfallen. Eine detektierbare Schadstoffkonzentration und eine Art von Schadstoffen, welche durch das Verfahren zur Wasserqualitätsüberwachung detektierbar sind, sind abhängig von einer gewählten Art von lebenden Testorganismen 40a. Reflektionen an den Wandelementen 14a, 16a, 18a, 20a werden ebenfalls in dem Signalspektrum als Amplitudenspitzen sichtbar, welche allerdings aufgrund einer bekannten Frequenzlage, da die Wandelemente 14a, 16a, 18a, 20a unbeweglich gegenüber einer Position der Schallsensoreinheit 22a sind, in einer Auswertung leicht identifiziert und ausgeschlossen werden.

Die Eigenbewegungen der lebenden Testorganismen 40a werden mittels Ultraschallmessung überwacht. Die Anregungssignale 32a, die von der Schallerregereinheit 28a ausgesandt werden, sind von Signalen im Ultraschall-Frequenzbereich mit einer Frequenz von 10 MHz gebildet. Auch die als Schalldopplersignale ausgebildeten Schallsignale 30a sind im Ultraschall-Frequenzbereich angesiedelt, da die Dopplerverschiebung zu gering ist, um eine Verschiebung in einen für Menschen hörbaren Bereich zu bewirken. Die lebenden Testorganismen 40a sind dahingehend ausgewählt, dass Ultraschall für sie unhörbar ist und werden daher durch die Anregungssignale 32a und/oder die Schallsignale 30a nicht beeinflusst.

Die gemessenen Schallsignale 30a werden in dem ersten Schallsensorelement 24a in Stromsignale umgewandelt und über das Datenübertragungselement 42a zu der Signalwandlungseinheit 36a gesendet, wo eine Differenzbildung mit einer Grundfrequenz, mit der die Signalgeneratoreinheit 34a die Schallerregereinheit 28a antreibt, durchgeführt wird, so dass nur die Dopplerverschiebung der Schallsignale 30a betrachtet wird, und anschließend eine Frequenz-Spannungswandlung erfolgt. Von der Signalwandlungseinheit 36a wird nach der Frequenz-Spannungswandlung ein Amplitudensignal an die Auswerteeinheit 38a gesendet. Grundsätzlich kann auch auf die Signalwandlungseinheit 36a verzichtet werden und die gemessenen Schallsignale 30a als Stromsignale direkt an die Auswerteeinheit 38a gesendet und dort ausgewertet werden. Das Amplitudensignal der Signalwandlungseinheit 36a und somit die gemessenen Schallsignale 30a werden in der Auswerteeinheit 38a mittels eines Filterverfahrens ausgewertet, bei dem mittels eines digitalen Filters in der Auswerteeinheit 38a ein erster Amplitudenbereich, der Frequenzen von Schalldopplersignalen von sich bewegenden lebenden Testorganismen 40a, die Fluchtbewegungen durchführen, entspricht, ein zweiter Amplitudenbereich, der Frequenzen von Schalldopplersignalen von Kiemenflossenbewegungen bei hektischer Atmung der lebenden Testorganismen 40a entspricht, und ein dritter Amplitudenbereich, welcher regungslosen lebenden Testorganismen 40a und somit insbesondere durch eine Schadstoffbelastung umgekommenen Testorganismen 40a entspricht, betrachtet werden. Die Auswerteeinheit 38a ist dazu programmiert, ein Alarmsignal, das vor einer Schadstoffbelastung warnt, auszugeben, wenn Amplituden in einem der drei Amplitudenbereiche eine vordefinierte Zeitspanne lang ununterbrochen einen Schwellenwert überschreiten, wodurch eine Anzahl von Fehlalarmen durch Zufallsereignisse, bei denen Amplitudensignale aus einem der drei Amplitudenbereiche entstehen, zu verringern.

In einer alternativen Ausgestaltung des erfindungsgemäßen Verfahrens können die gemessenen Schallsignale 30a mittels eines numerischen Verfahrens ausgewertet werden. Beispielsweise wird mittels einer Fast-Fourier-Transformation das Signalspektrum aus den gemessenen Schallsignalen 30a erstellt. Anstelle einer Fast-Fourier-Transformation kann in weiteren alternativen Ausgestaltungen ein anderes numerisches Verfahren wie beispielsweise eine Wavelet-Analyse angewandt werden. Das mit dem numerischen Verfahren erstellte Signalspektrum wird mittels eines Filterverfahrens ausgewertet, bei dem mittels eines digitalen Filters in der Auswerteeinheit 38a das Signalspektrum in einem ersten Frequenzbereich, der Frequenzen von Schalldopplersignalen von sich bewegenden lebenden Testorganismen 40a, die Fluchtbewegungen durchführen, umfasst und in einem zweiten Frequenzbereich, der Frequenzen von Schalldopplersignalen von Kiemenflossenbewegungen bei hektischer Atmung der lebenden Testorganismen 40a umfasst, betrachtet wird. In einer alternativen Ausgestaltung kann anstelle eines digitalen Filters auch ein analoges Filter verwendet werden, welches Signale, die Frequenzen außerhalb des ersten Frequenzbereichs und des zweiten Frequenzbereichs aufweisen, teilweise oder vollständig abschwächt, bevor sie in der Auswerteeinheit 38a verarbeitet werden.

In einer Ausbildung des Verfahrens wird ein Signalspektrum gemessener Schallsignale 30a mit einem Referenzspektrum für eine Bewegung der lebenden Testorganismen 40a bei Normalbedingungen verglichen. Eine Erstellung des Signalspektrums aus den gemessenen Schallsignalen 30a und ein Vergleich mit dem Referenzspektrum wird durch die Auswerteeinheit 38a bewirkt. Liegt eine Abweichung von Amplitudenhöhe und Frequenz von Amplitudenspitzen des Signalspektrums und des Referenzspektrums über einem definierten und in einer Speichereinheit der Auswerteeinheit 38a hinterlegten Schwellenwert, so wird von der Auswerteeinheit 38a ein Signal ausgegeben, dass eine Schadstoffbelastung festgestellt wurde.

In einer weiteren Variante des Teilverfahrens werden über einen Meßzeitraum gemessene Signalspektren miteinander verglichen. Der Meßzeitraum kann eine feste, voreingestellte Zeitdauer sein, während der die Signalspektren miteinander verglichen werden, und nach deren Ablauf ein Auswerteergebnis ausgegeben wird, oder der Meßzeitraum kann von einem manuell oder fernbedient eingestellten Startzeitpunkt an laufen, bis er durch ein manuell oder fernbedient eingestelltes Endsignal abgebrochen wird, wobei während des Meßzeitraums ein Warnsignal ausgesandt wird, wenn die Auswerteeinheit 38a eine Überschreitung Schadstoffbelastung feststellt, und/oder nach Beendigung des Meßzeitraums ein Auswerteergebnis ausgegeben wird. Bevorzugt beginnt der Meßzeitraum kurz nach einem Einsetzen der lebenden Testorganismen 40a in das Testbehältnis 12a, so dass diese anfangs in einem ruhigen Zustand sind. Wird von der Auswerteeinheit 38a eine Verschiebung der Amplitudenspitzen der Signalspektren im Verlauf des Meßzeitraums zu höheren Frequenzen und eine Zunahme einer Amplitude verschobener Amplitudenspitzen über definierten Schwellenwerten festgestellt, die auf ein hektisches Verhalten der lebenden Testorganismen 40a und somit eine Schadstoffbelastung hinweisen, so wird von der Auswerteeinheit 38a ein Signal über die Schadstoffbelastung ausgegeben.

In einer Ausgestaltung des Verfahrens wird ein Normalverhaltenssignal der lebenden Testorganismen dynamisch aus gemessenen Schallsignalen 30a bestimmt. In einem vordefinierten Zeitraum werden spannungsgewandelte Signale der gemessene Schallsignale 30a mittels eines Tiefpassfilterverfahrens bearbeitet, so dass hochfrequente Schallsignale 30a, die rapiden Eigenbewegungen der lebenden Testorganismen 40a entsprechen, während des vordefinierten Zeitraums bei der Auswertung abgeschwächt werden und hauptsächlich niederfrequente Schallsignale 30a, welche ruhigen Eigenbewegungen der lebenden Testorganismen 40a entsprechen, im Wesentlichen unabgeschwächt durchgelassen werden. Die Spannungssignale nach Durchführung des Filterverfahrens werden anschließend über den Zeitraum aufintegriert und ein Mittelwert hieraus gebildet, welcher als Normalverhaltenssignal genutzt wird. Grundsätzlich kann die Mittelwertbildung und Bestimmung des Normalverhaltenssignals mehrfach, bevorzugt in regelmäßigen Abständen, wiederholt werden.

Die Schallerregereinheit 28a umfasst ein piezoelektrisches Element, das in den Hohlraum des Testbehältnisses 12a ragt und durch die Signalgeneratoreinheit 34a mit einer Frequenz in einem Frequenzbereich von 500 kHz bis 20 MHz begetrieben wird, wodurch Anregungssignale 32a in einem Ultraschall-Frequenzbereich ausgesandt werden (Fig. 2). Die Anregungssignale 32a werden von der Schallerregereinheit 28a in einem Öffnungswinkel von 15° Grad gerichtet abgestrahlt. Grundsätzlich könnte die Schallerregereinheit 28a auch eine ungerichtete Abstrahlcharakteristik mit einem weitgehend raumfüllenden Öffnungswinkel aufweisen. Die Anregungssignale 32a zu einer Erzeugung von Schalldopplersignalen werden in einem kontinuierlichen Betrieb eingestrahlt, auch wird die Schallmessung kontinuierlich durchgeführt. Eigenbewegungen der lebenden Testorganismen 40a werden somit ständig überwacht. Alternativ können die Anregungssignale 32a zu der Erzeugung von Schalldopplersignalen in einem diskontinuierlichen Betrieb eingestrahlt werden, in dem zu definierten Zeitpunkten, unterbrochen von Perioden, in denen die Schallerregereinheit 28a inaktiv ist, Anregungssignale 32a eingestrahlt werden. Bei einem solchen Betriebsverfahren wird ein verringerter Überwachungsaufwand erreicht, was besonders bei Langzeitmessungen vorteilhaft ist. In dem diskontinuierlichen Betrieb kann die Schallerregereinheit 28a einstückig mit dem Schallsensorelement 24a ausgeführt sein, wobei ein Piezoelement zwischen einem Sendemodus und einem Empfangsmodus umgeschaltet wird.

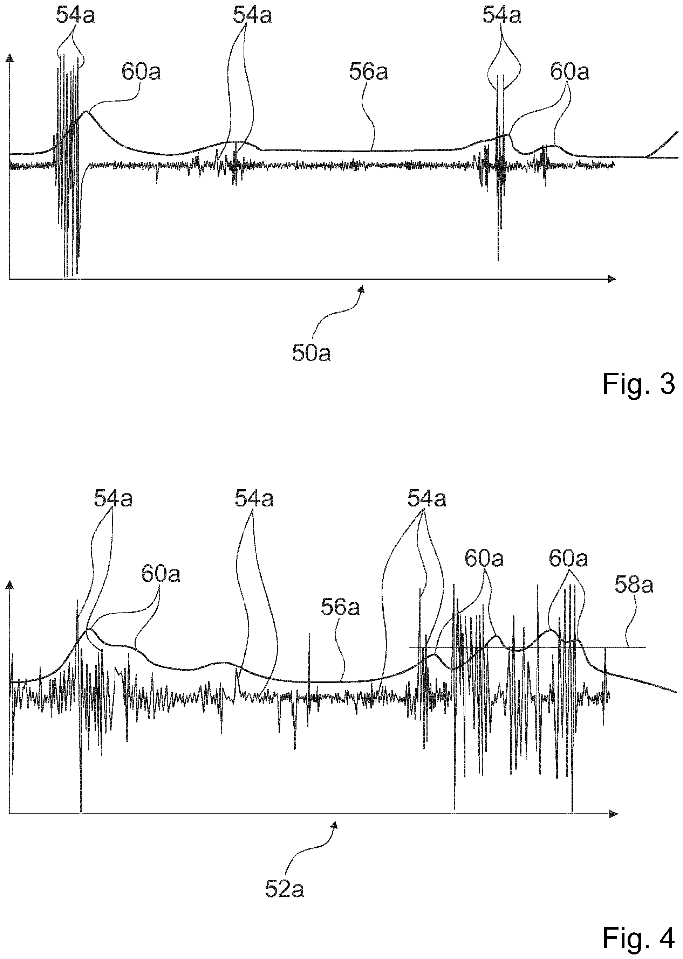

Fig. 3 zeigt ein Amplitudendiagramm 50a mit Amplitudensignalen 54a, das mittels der Signalwandlungseinheit 34a aus gemessenen Schallsignalen 30a erstellt und in der Auswerteeinheit 38a ausgewertet wird. Die gemessenen Schallsignale 30a wurden dabei mittels Frequenz-Spannungswandlung in Amplitudensignale 54a einer Spannung umgewandelt. Das Amplitudendiagramm 50a ist charakteristische für weitgehend ruhiges Verhalten der lebenden Testorganismen 40a. Eine Höhe der Amplitudensignale 54a entspricht einer Dopplerfrequenzverschiebung eines empfangenen Schallsignals 30a gegenüber dem Anregungssignal 32a, wobei eine positive und negative Spannung jeweils eine Richtung der Dopplerverschiebung angeben und somit, ob sich die lebenden Testorganismen 40a oder sich bewegende Körperteile an ihnen in Richtung auf das Schallsensorelement 24a zubewegt oder von ihm wegbewegt hat. Ein überwiegender Anteil der Amplitudensignalen 54a weist nur eine geringe Höhe auf, so dass sich erkennen lässt, dass die lebenden Testorganismen 40a sich überwiegend ruhig bewegt haben. Eine Auswertung des Amplitudendiagramms 50a geschieht durch ein Filterverfahren, bei dem lediglich Amplitudensignale 54a mit einer Höhe über einen Schwellenwert betrachtet werden, und zusätzlich ausgewertet wird, wie häufig über einen Zeitraum Amplitudensignale 54a mit der Höhe über dem Schwellenwert auftreten, um einen Fehlalarm aufgrund eines Einzelereignisses zu vermeiden. Alternativ kann ein Tiefpass-Filterverfahren verwendet werden, das Frequenzen oberhalb einer Grenzfrequenz abschwächt. Steigt eine Amplitude eines gefilterten Signals bei Tiefpass-Filterung an, so entspricht dies dem hektischen Zustand der lebenden Testorganismen 40a.

Ferner ist in Fig. 3 ein beispielhaftes Filtersignal 56a, das aus gemessenen Schallsignalen 30a erzeugt wurde, dargestellt. Das Filtersignal 56a wurde mittels eines Tiefpassfilterverfahrens aus den spannungsgewandelten Signalen der Schallsignale 30a erzeugt. Bei einer Zunahme von Geschwindigkeit und Häufigkeit von Eigenbewegungen der lebenden Testorganismen 40a zeigt das Filtersignal 56a Filtersignalspitzen 60a, die im Vergleich gegenüber Amplitudenspitzen der Amplitudensignale 54a geringer ausfallen.

Fig. 4 zeigt ein weiteres Amplitudendiagramm 52a mit Amplitudensignalen 54a, das einem hektischen Zustand der lebenden Testorganismen 40a entspricht und somit charakteristisch ist für ein Messergebnis bei einem Vorliegen einer Schadstoffbelastung. Ein Großteil der Amplitudensignale 54a weist eine Höhe über dem Schwellenwert auf, was schnellen Eigenbewegungen der lebenden Testorganismen 40a und somit einem hektischen Verhalten entspricht. Ebenfalls ist ein entsprechendes, beispielhaftes Filtersignal 56a dargestellt. Liegen die Filtersignalspitzen 60a über einem Schwellenwert 58a, welcher in der Auswerteeinheit 38a hinterlegt ist oder dynamisch von der Auswerteeinheit 38a bestimmt wird, so wird ein Alarmsignal ausgelöst, das auf eine wahrscheinliche Schadstoffbelastung hinweist.

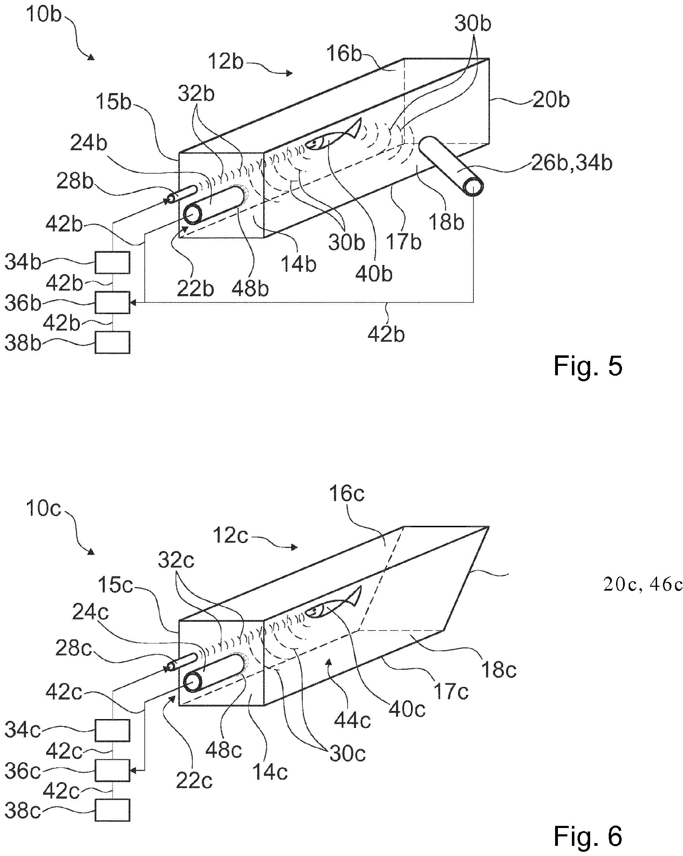

In den Figuren 5 bis 9 sind vier weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 4, verwiesen wird. Zur Unterscheidung der Ausführungsbeispiele ist der Buchstabe a den Bezugszeichen des Ausführungsbeispiels in den Figuren 1 bis 4 nachgestellt. In den Ausführungsbeispielen der Figuren 5 bis 9 ist der Buchstabe a durch die Buchstaben b bis f ersetzt.

Fig. 5 zeigt eine Vorrichtung 10b zur Durchführung eines Verfahrens zur Wasserqualitätsüberwachung mittels einer Überwachung von Eigenbewegungen lebender Testorganismen 40b in einem Wasservolumen 44b, wobei die Eigenbewegungen der lebenden Testorganismen 40b mittels Schallmessung überwacht werden, mit einem Testbehältnis 12b für die lebenden Testorganismen 40b, das als verschließbarer Wassertank mit sechs Wandelementen 14b, 15b, 16b, 17b, 18b, 20b, einer Schallsensoreinheit 22b mit einem ersten Schallsensorelement 24b, das einen Piezo-Wandler aufweist, einer Schallerregereinheit 28b, einer Signalgeneratoreinheit 34a, einer Signalwandlungseinheit 36a und einer Auswerteeinheit 38b, die durch die Schallsensoreinheit 22b gemessene Schallsignale 30b auswertet. Die Vorrichtung 10b entspricht im Wesentlichen der des ersten Ausführungsbeispiels. Das erste Schallsensorelement 24b ist zu einer Aufnahme von Schallsignalen 30b entlang einer ersten Raumrichtung des Testbehältnisses 12b vorgesehen. Die erste Raumrichtung ist dabei von einer Richtung einer größten Längserstreckung des Testbehältnisses 12b gebildet. Die Schallsensoreinheit 22b der Vorrichtung 10b weist zudem ein zweites Schallsensorelement 26b zu einer Aufnahme von Schallsignalen 30b entlang einer zweiten Raumrichtung des Testbehältnisses 12b auf. Die zweite Raumrichtung ist von einer senkrecht zu der ersten Raumrichtung verlaufenden Richtung gebildet. Durch das erste Schallsensorelement 24b und das zweite Schallsensorelement 26b, die Schallsignale 30b entlang unterschiedlicher Raumrichtungen aufnehmen, wird ein hoher Erfassungsgrad von Schallsignalen 30b erreicht. Das erste Schallsensorelement 24b und das zweite Schallsensorelement 26b sind jeweils von einem Schutzelement 48b umgeben, welches Beschädigungen und Fehlsignale des ersten Schallsensorelements 24b und des zweiten Schallsensorelements 26b, die durch Verbiss des ersten Schallsensorelements 24b oder des zweiten Schallsensorelements 26b durch die lebenden Testorganismen 40b auftreten können, verhindert. Für eine Auswertung der Eigenbewegungen der lebenden Testorganismen 40b werden die von dem ersten Schallsensorelement 24b und dem zweiten Schallsensorelement 26b gemessenen Schallsignale 30b an eine Signalwandlungseinheit 36b zu einer Differenzbildung mit einer Grundfrequenz, mit der die Signalgeneratoreinheit 34b die Schallerregereinheit 28b antreibt und einer anschließenden Frequenz-Spannungswandlung gesendet, von der ein Amplitudensignal an die Auswerteeinheit 38b gesendet wird. Dieses Amplitudensignal wird mittels eines Filterverfahrens ausgewertet, bei dem mittels eines digitalen Filters in der Auswerteeinheit 38b ein erster Amplitudenbereich, der Frequenzen von Schalldopplersignalen von sich bewegenden lebenden Testorganismen 40b, die Fluchtbewegungen durchführen, entspricht, ein zweiter Amplitudenbereich, der Frequenzen von Schalldopplersignalen von Kiemenflossenbewegungen bei hektischer Atmung der lebenden Testorganismen 40b entspricht, und einer dritter Amplitudenbereich, welcher regungslosen lebenden Testorganismen 40b und somit insbesondere durch eine Schadstoffbelastung umgekommenen Testorganismen 40b entspricht, betrachtet werden. Alternativ können die von dem ersten Schallsensorelement 24b und dem zweiten Schallsensorelement 26b gemessenen Schallsignale 30b mittels eines numerischen Verfahrens ausgewertet, werden indem mittels einer Fast-Fourier-Transformation jeweils ein separates Signalspektrum erstellt wird. Anschließend werden die separaten Signalspektren mittels Vergleich mit Referenzspektren, einem Vergleich mit weiteren Signalspektren während eines Meßzeitraums und/oder mittels eines Filterverfahrens ausgewertet. In einer alternativen Auswertung wird ein spannungsgewandeltes Frequenzsignal direkt ausgewertet.

Eine zweite alternative Ausgestaltung einer Vorrichtung 10c zur Durchführung eines Verfahrens zur Wasserqualitätsüberwachung mittels einer Überwachung von Eigenbewegungen lebender Testorganismen 40c in einem Wasservolumen 44c, wobei die Eigenbewegungen der lebenden Testorganismen 40c mittels Schallmessung überwacht werden, ist in Fig. 6 dargestellt. Die Vorrichtung 10c umfasst ein Testbehältnis 12c für die lebenden Testorganismen 40c mit sechs Wandelementen 14c, 15c, 16c, 17c, 18c, 20c, eine Schallsensoreinheit 22c ausgebildeten ersten Schallsensorelement 24c, das einen Piezo-Wandler aufweist, einer Schallerregereinheit 28c und einer Auswerteeinheit 38c, die durch die Schallsensoreinheit 22c gemessene Schallsignale 30c auswertet. Die Auswerteeinheit 38c ist über von Kabeln gebildete Datenübertragungselemente 42c mit einer Signalwandlungseinheit 36c verbunden. Alternativ können die Datenübertragungselemente 42c auch eine drahtlose Verbindung bereitstellen. Die Schallerregereinheit 28c wird von einer Signalgeneratoreinheit 34c angetrieben. Das Testbehältnis 12c für lebende Testorganismen 40c ist von einem verschließbaren Wassertank gebildet, der massive Wandelemente 14c, 16c, 18c, 20c aus Glas aufweist, welche einen Hohlraum, in welchen das Wasservolumen 44c mit den darein gesetzten lebenden Testorganismen 40c aufgenommen ist, begrenzen. Der verschließbare Wassertank weist eine Schrägwand 46c zu einer Umlenkung von Schallsignalen 30c auf, wobei das Wandelement 18c als die Schrägwand 46c ausgebildet ist. Die Schrägwand 46c ist als rechteckiges Wandelement 18c ausgebildet, welches in dem Ausführungsbeispiel in einem Winkel von 45° zu den benachbarten Wandelementen 16c, 20c angeordnet ist. Andere Winkelstellungen zu den benachbarten Wandelementen 16c sind ebenfalls möglich. Anregungssignale 32c und Schallsignale 30c werden durch die Schrägwand 46c reflektiert, wobei durch den Winkel der Schrägwand 46c von 45° zu den benachbarten Wandelementen 16c, 20c die reflektierten Anregungssignale 32c und Schallsignale 30c unter einem Winkel von 45° zu Haupterstreckungsrichtungen des Hohlraums reflektiert werden, wodurch ein hoher Erfüllungsgrad des Wasservolumens 44c und der lebenden Testorganismen 40c in dem Hohlraum durch die Anregungssignale 32c, nicht dopplerverschobene reflektierte Anregungssignale 32c und an den lebenden Testorganismen 40c reflektierte Schallsignale 30c erreicht wird.

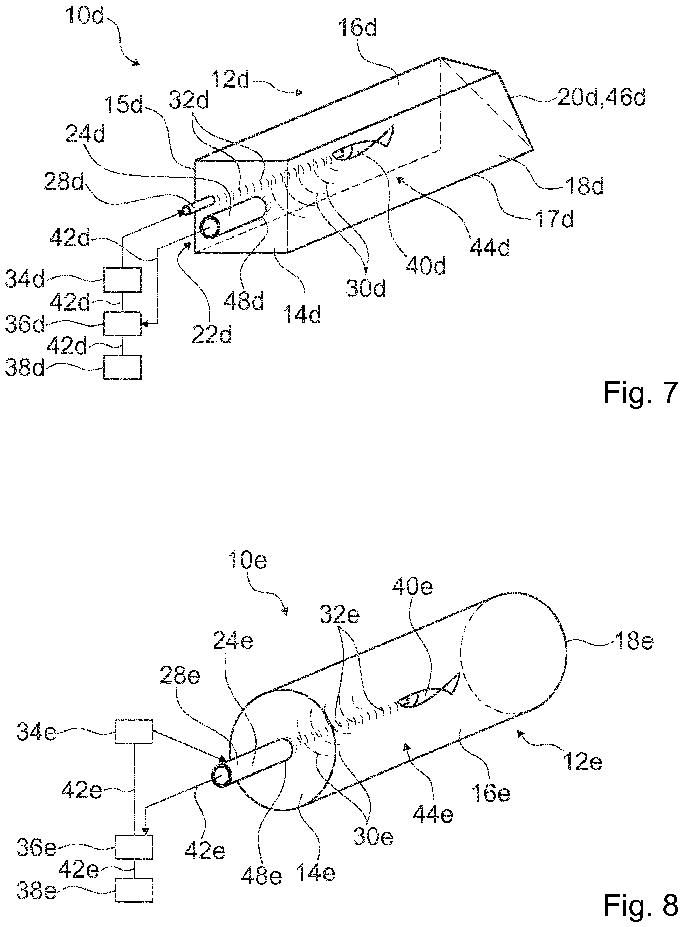

Eine weitere Variante einer Vorrichtung 10d zur Durchführung eines Verfahrens zur Wasserqualitätsüberwachung mittels einer Überwachung von Eigenbewegungen lebender Testorganismen 40d in einem Wasservolumen 44d, wobei die Eigenbewegungen der lebenden Testorganismen 40d mittels Schallmessung überwacht werden, mit einem Testbehältnis 12d für die lebenden Testorganismen 40d mit sechs Wandelementen 14d, 15d, 16d, 17d, 18d, 20d, einer Schallsensoreinheit 22d mit einem ersten Schallsensorelement 24d, das einen Piezo-Wandler aufweist, einer Schallerregereinheit 28d und einer Auswerteeinheit 38d, die durch die Schallsensoreinheit 22d gemessene Schallsignale 30d auswertet, wobei ein Wandelement 18d als Schrägwand 46d ausgebildet ist, ist in Fig. 7 dargestellt. Die Schrägwand 46d besteht aus zwei dreieckigen Teilwänden, die in einem Winkel von 45° zueinander angeordnet sind. Die Schrägwand 46d ist somit doppelt geneigt. Aufgrund einer Neigung der Schrägwand 46d gegenüber den anderen Wandelementen 14d, 16d, 20d werden die Anregungssignale 32d reflektiert, so dass eine hohe Ausfüllung eines Wasservolumens 44d durch Anregungssignale 32d erreicht wird.

Fig. 8 zeigt eine vierte, alternative Ausbildung einer Vorrichtung 10e zur Durchführung eines Verfahrens zur Wasserqualitätsüberwachung mittels einer Überwachung von Eigenbewegungen lebender Testorganismen 40e in einem Wasservolumen 44e, wobei die Eigenbewegungen der lebenden Testorganismen 40e mittels Schallmessung überwacht werden. Die Vorrichtung 10e umfasst ein Testbehältnis 12e für die lebenden Testorganismen 40e mit drei Wandelementen 14e, 16e, 18e, einer Schallsensoreinheit 22e mit einem ersten Schallsensorelement 24e, das ein Piezoelement aufweist und das mit einer Schallerregereinheit 28e einstückig ausgeführt ist, und einer Auswerteeinheit 38e, die durch die Schallsensoreinheit 22e gemessene Schallsignale 30e auswertet. In einem kontinuierlichen Betrieb wird das Piezoelement von einem Sendemodus in einen Empfangsmodus und umgekehrt umgeschaltet und sendet so in einem steten Wechsel Anregungssignale 30e aus und sensiert auftreffende Schallsignale 30e. Das Testbehältnis 12e für lebende Testorganismen 40e ist von einem verschließbaren Wassertank gebildet, der massive Wandelemente 14e, 16e, 18e, aus Glas aufweist, welche einen Hohlraum, in welchen das Wasservolumen 44e mit den eingesetzten lebenden Testorganismen 40e aufgenommen ist, begrenzen, und weist eine Zylinderform mit zwei kreisförmigen Wandelementen 14e, 18e und einem als Mantelfläche ausgebildeten Wandelement 16e auf. Alternativ können die Wandelemente 14e, 16e, 18e teilweise oder vollständig aus Kunststoff, Glas oder Metall bestehen.

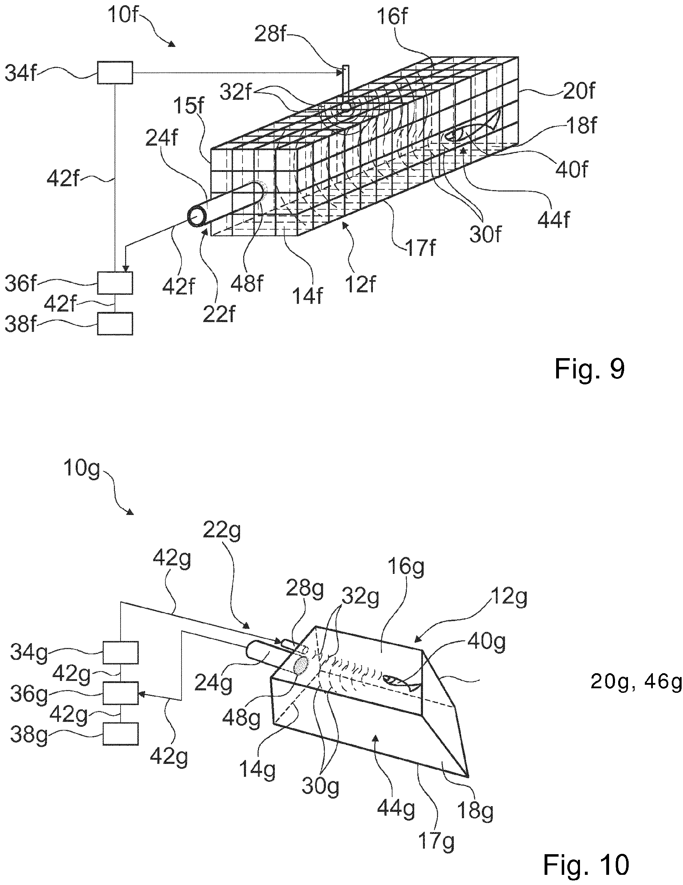

Fig. 9 zeigt ein fünftes, alternatives Ausführungsbeispiel einer Vorrichtung 10f zur Durchführung eines Verfahrens zur Wasserqualitätsüberwachung mittels einer Überwachung von Eigenbewegungen lebender Testorganismen 40f in einem Wasservolumen 44f, wobei die Eigenbewegungen der lebenden Testorganismen 40f mittels Schallmessung überwacht werden. Die Vorrichtung 10f umfasst ein Testbehältnis 12f für die lebenden Testorganismen 40f mit sechs Wandelementen 14f, 15f, 16f, 17f, 18f, 20f, einer Schallsensoreinheit 22f mit einem ersten Schallsensorelement 24f, das einen Piezo-Wandler aufweist, einer Schallerregereinheit 28f und einer Auswerteeinheit 38f, die durch die Schallsensoreinheit 22f gemessene Schallsignale 30f auswertet. Die Schallerregereinheit 28f strahlt Anregungssignale 32f in einem kontinuierlichen Betrieb mit einem Öffnungswinkel von 2π aus, so dass ein Rauminhalt des Testbehältnisses 12f annähernd vollständig mit Anregungssignalen 32f ausgefüllt wird. Das Testbehältnis 12f für lebende Testorganismen 40f ist als Gitterkäfig mit metallenen Gitterstäben ausgebildet, wobei ein Abstand der Gitterstäbe untereinander so gewählt ist, dass die lebenden Testorganismen 40f in einem von den Wandelementen 14f, 15f, 16f, 17f, 18f, 20f begrenzten Hohlraum eingeschlossen sind. Das Testbehältnis 12f weist ferner eine nicht dargestellte Klappe auf, durch die die lebenden Testorganismen 40f in den Hohlraum eingeführt werden können. Das Testbehältnis 12f wird in ein Wasser, das auf eine Schadstoffbelastung hin untersucht werden soll, beispielsweise ein natürliches Gewässer wie ein Bach oder ein Fluss oder eine künstliche Wasserleitung, eingesetzt und anschließend mit den lebenden Testorganismen 40f besetzt. Diese Ausgestaltung der Erfindung ist besonders für eine Langzeitüberwachung einer Wasserqualität geeignet, da die lebenden Testorganismen 40a zum einem stets mit Frischwasser versorgt werden und zum anderen natürlich vorkommendes Futter, welches durch die Gitterstäbe in das Testbehältnis 12f eintritt, wie beispielsweise Kleintiere oder Pflanzenteile, aufnehmen können, so dass gegebenenfalls eine Fütterung der lebenden Testorganismen 40a entfallen kann. Fig. 10 zeigt eine sechste Variante einer Vorrichtung 10g zur Durchführung eines Verfahrens zur Wasserqualitätsüberwachung mittels einer Überwachung von Eigenbewegungen lebender Testorganismen 40g in einem Wasservolumen 44g, wobei die Eigenbewegungen der lebenden Testorganismen 40g mittels Schallmessung überwacht werden. Die Vorrichtung 10g umfasst ein Testbehältnis 12g für die lebenden Testorganismen 40g mit sechs Wandelementen 14g, 16g, 17g, 18g, 20g, eine Schallsensoreinheit 22g mit ersten Schallsensorelement 24g, das einen Piezo-Wandler aufweist, eine Schallerregereinheit 28g und eine Auswerteeinheit 38g, die durch die Schallsensoreinheit 22g gemessene Schallsignale 30g auswertet. Eines der Wandelemente 20g ist als zweifach verkippte Schrägwand 46g ausgebildet, die gegenüber dem gegenüberliegenden Wandelement 14g um neunzig Grad gedreht und zusätzlich schräg geneigt ist.

### Bezugszeichen

- 10: Vorrichtung
- 12: Testbehältnis
- 14: Wandelement
- 15: Wandelement
- 16: Wandelement
- 17: Wandelement
- 18: Wandelement
- 20: Wandelement
- 22: Schallsensoreinheit
- 24: Schallsensorelement
- 26: Schallsensorelement
- 28: Schallerregereinheit
- 30: Schallsignal
- 32: Anregungssignal
- 34: Signalgeneratoreinheit
- 36: Signalwandlungseinheit
- 38: Auswerteeinheit
- 40: Testorganismus
- 42: Datenübertragungselement
- 44: Wasservolumen
- 46: Schrägwand
- 48: Schutzelement
- 50: Amplitudendiagramm
- 52: Amplitudendiagramm
- 54: Amplitudensignal
- 56: Filtersignal
- 58: Schwellenwert
- 60: Filtersignalspitze

## Patentansprüche

1. Verfahren zur Wasserqualitätsüberwachung mittels zumindest einer Überwachung von Eigenbewegungen lebender Testorganismen (40a-g), insbesondere Fischen und/oder Daphnien, in einem Wasservolumen (44ag), wobei über einen Meßzeitraum gemessene Schallsignale (30a-g) miteinander verglichen werden, wobei aus einer Veränderung der Signale über den Meßzeitraum eine Verhaltensänderung der lebenden Testorganismen (40a-g) während eines Verlaufs des Meßzeitraums bestimmt wird, **dadurch gekennzeichnet, dass** die Eigenbewegungen der lebenden Testorganismen (40a-g) mittels Schalldopplermessung überwacht werden, wobei eine Zunahme oder Abnahme einer Hektik aufgrund einer im Verlauf des Meßzeitraums zunehmenden oder abnehmenden Frequenzverschiebung der Schallsignale bestimmt wird und wobei eine erhöhte Atemfrequenz durch eine Intensität und Frequenz einer einer Kiemenflossenbewegung zugeordneten Amplitudenspitze in einem als Dopplerspektrum ausgebildeten Signalspektrum der Schallsignale (30a-g) detektiert wird.

2. Verfahren nach Anspruch 1, **dadurch gekennzeichnet, dass** die Eigenbewegungen der lebenden Testorganismen (40a-g) mittels Ultraschalldopplermessung überwacht werden.

3. Verfahren nach einem der vorhergehenden Ansprüche, **dadurch gekennzeichnet, dass** gemessene Schallsignale (30a-g) mit zumindest einem Referenzsignal für eine Eigenbewegung der lebenden Testorganismen (40a-g) bei Normalbedingungen verglichen werden.

4. Verfahren nach Anspruch 3, **dadurch gekennzeichnet, dass** mehrere Referenzsignale für Normalverhalten für unterschiedliche Zeitpunkte verwendet werden.

5. Verfahren nach einem der vorhergehenden Ansprüche, **dadurch gekennzeichnet, dass** die gemessenen Schallsignale (30a-g) mittels eines numerischen Verfahrens ausgewertet werden.

6. Verfahren nach einem der vorhergehenden Ansprüche, **dadurch gekennzeichnet, dass** die gemessenen Schallsignale (30a-g) mittels eines Filterverfahrens ausgewertet werden.

7. Verfahren nach einem der vorhergehenden Ansprüche, **dadurch gekennzeichnet, dass** ein Normalverhaltenssignal der lebenden Testorganismen (40a-g) dynamisch aus gemessenen Schallsignalen (30ag) bestimmt wird.

8. Verfahren zumindest nach Anspruch 1, **dadurch gekennzeichnet, dass** Anregungssignale (32a-g) zu einer Erzeugung von Schalldopplersignalen in einem kontinuierlichen Betrieb eingestrahlt werden.

9. Verfahren zumindest nach Anspruch 1, **dadurch gekennzeichnet, dass** Anregungssignale (32a-g) zu einer Erzeugung von Schalldopplersignalen in einem diskontinuierlichen Betrieb eingestrahlt werden.

10. Vorrichtung (10a-g), mit zumindest einem Testbehältnis (12a-g) für lebende Testorganismen (40a-g), zumindest einer Schallsensoreinheit (22a-d, f, g) mit einem ersten Schallsensorelement (24a-g), einer Schallerregereinheit (28a-g), einer Signalgeneratoreinheit (34ag), die dazu eingerichtet ist die Schallerregereinheit (28a-g) anzutreiben, und zumindest einer Auswerteeinheit (38a-g), die dazu eingerichtet ist die durch die Schallsensoreinheit (22a-d, f, g) gemessenen Schallsignale (30ag) auszuwerten, **dadurch gekennzeichnet, dass**
das Schallsensorelement (24a-g) einen Piezowandler aufweist und
die Vorrichtung (10a-g) dazu eingerichtet ist eines der Verfahren nach einem der vorhergehenden Ansprüche auszuführen.

11. Vorrichtung nach Anspruch 10, **dadurch gekennzeichnet, dass** das zumindest eine Testbehältnis (12a, c, d, g) für die lebenden Testorganismen (40a-e) von einem verschließbaren Wassertank gebildet ist.

12. Vorrichtung nach Anspruch 11, **dadurch gekennzeichnet, dass** der verschließbare Wassertank aus sechs Wandelementen (14c, d, g; 15c, d, g; 16c, d, g; 17c, d, g; 18c, d, g; 20c, d, g) besteht und zumindest eine Schrägwand (46cd; 46g), das heißt ein Wandelement des Wassertanks, das in einem von einem rechten Winkel verschiedenen Winkel zu zumindest einem weiteren Wandelement des Wassertanks angeordnet ist, zu einer Umlenkung von Schallsignalen (30c-d, 30g) aufweist.

13. Vorrichtung nach Anspruch 10, **dadurch gekennzeichnet, dass** das zumindest eine Testbehältnis (12f) für die lebenden Testorganismen (40f) von einem Gitterkäfig gebildet ist.

14. Vorrichtung nach einem der Ansprüche 10 bis 13, **dadurch gekennzeichnet, dass** die zumindest eine Schallsensoreinheit (22b) zumindest ein erstes Schallsensorelement (24b) zu einer Aufnahme von Schallsignalen (30b) entlang einer ersten Raumrichtung des zumindest einen Testbehältnisses (12b) und zumindest ein zweites Schallsensorelement (26b) zu einer Aufnahme von Schallsignalen (30b) entlang einer zweiten Raumrichtung des zumindest einen Testbehältnisses (12b) aufweist.

## Claims

1. Method for water quality monitoring by at least one monitoring of proper motions of living test organisms (40a-g), in particular fish and/or daphnia, in a water volume (44a-g), wherein acoustic signals (30a-g) measured over a measuring period are compared to one another, wherein a change in a behaviour of the living test organisms (40a-g) during a course of the measuring period is determined by a change in the signals over the measuring period,
**characterised in that** the proper motions of the living test organisms (40a-g) are monitored via Doppler sonography measuring, wherein an increase or decrease of an agitation is determined on the basis of a frequency shift of the acoustic signals that increases or decreases in the course of the measuring period and wherein an increased respiration frequency is detected via an intensity and frequency of a peak amplitude, allocated to a gill cover movement, in a signal spectrum of the acoustic signals (30a-g) that is implemented as a Doppler spectrum.

2. Method according to claim 1,
**characterised in that** the proper motions of the living test organisms (40a-g) are monitored via Doppler ultrasonography measuring.

3. Method according to one of the preceding claims,
**characterised in that** measured acoustic signals (30a-g) are compared to at least one reference signal for a proper motion of the living test organisms (40a-g) under normal conditions.

4. Method according to claim 3,
**characterised in that** a plurality of reference signals for normal behaviour are used for different points in time.

5. Method according to one of the preceding claims,
**characterised in that** the measured acoustic signals (30a-g) are evaluated via a numeric method.

6. Method according to one of the preceding claims,
**characterised in that** the measured acoustic signals (30a-g) are evaluated via a filter method.

7. Method according to one of the preceding claims,
**characterised in that** a signal for normal behaviour of the living test organisms (40a-g) is determined dynamically on the basis of measured acoustic signals (30a-g).

8. Method at least according to claim 1,
**characterised in that** excitation signals (32a-g) for a generation of Doppler sound signals are irradiated in a continued operation.

9. Method at least according to claim 1,
**characterised in that** excitation signals (32a-g) for a generation of Doppler sound signals are irradiated in a discontinued operation.

10. Device (10a-g), with at least one test container (12a-g) for living test organisms (40a-g), with at least one acoustic sensor unit (22a-d, f, g) with a first acoustic sensor element (24a-g), with a sound excitator unit (28a-g), with a signal excitator unit (34a-g) configured to drive the sound excitator unit (28a-g), and with at least one evaluation unit (38a-g) configured to evaluate the acoustic signals (30a-g) measured by the acoustic sensor unit (22a-d, f, g),
**characterised in that** the acoustic sensor element (24a-g) comprises a piezoelectric converter and the device (10a-g) is configured to implement one of the methods according to one of the preceding claims.

11. Device according to claim 10,
**characterised in that** the at least one test container (12a, c, d, g) for the living test organisms (40a-g) is embodied by a lockable water tank.

12. Device according to claim 11,
**characterised in that** the lockable water tank consists of six wall elements (14c, d, g; 15c, d, g; 16c, d, g; 17c, d, g; 18c, d, g; 20c, d, g) and comprises, for the purpose of deflecting acoustic signals (30c-d, 30g), at least one inclined wall (46c-d; 46g), i.e. a wall element of the water tank that is arranged at an angle, differing from a right angle, to at least one further wall element of the water tank.

13. Device according to claim 10,
**characterised in that** the at least one test container (12f) for the living test organisms (40f) is implemented by a mesh cage.

14. Device according to one of claims 10 to 13,
**characterised in that** the at least one acoustic sensor unit (22b) comprises at least one first acoustic sensor element (24b) for the purpose of recording acoustic signals (30b) along a first spatial direction of the at least one test container (12b) and comprises at least one second sensor element (26b) for the purpose of recording acoustic signals (30b) along a second spatial direction of the at least one test container (12b).

## Revendications

1. Procédé de surveillance de la qualité de l'eau par le biais d'au moins une surveillance des mouvements propres d'organismes d'essai vivants (40a-g), notamment des poissons et/ou des daphnies, dans un volume d'eau (44a-g), dans lequel procédé des signaux acoustiques (30a-g) mesurés pendant une période de mesure sont mutuellement comparés, un change de comportement des organismes d'essai vivants (40a-g) étant déterminé, pendant la période de mesure, sur la base d'un change des signaux au course de la période de mesure,
**caractérisé en ce que** les mouvements propres des organismes d'essai vivants (40a-g) sont surveillés par mesurage par sonographie Doppler, une croissance ou diminution d'une agitation étant déterminée sur la base d'un décalage de fréquence des signaux acoustiques croissant ou diminuant au cours de la période de mesure et une fréquence de respiration augmentée étant détectée, dans un spectre des signaux acoustiques (30a-g) implémenté comme spectre Doppler, par une intensité et fréquence d'une amplitude de crête allouée à un mouvement des opercules des branchies.

2. Procédé selon la revendication 1,
**caractérisé en ce que** les mouvements propres des organismes d'essai vivants (40a-g) sont surveillés par échographie Doppler.

3. Procédé selon l'une quelconque des revendications précédentes,
**caractérisé en ce que** des signaux acoustiques (30a-g) mesurés sont comparés à au moins un signal de référence pour un mouvement propre des organismes d'essai vivants (40a-g) dans des conditions normales.

4. Procédé selon la revendication 3,
**caractérisé en ce que** plusieurs signaux de référence pour un comportement normal sont utilisés pour des instants différents.

5. Procédé selon l'une quelconque des revendications précédentes,
**caractérisé en ce que** les signaux acoustiques (30a-g) mesurés sont évalués selon une méthode numérique.

6. Procédé selon l'une quelconque des revendications précédentes,
**caractérisé en ce que** les signaux acoustiques (30a-g) mesurés sont évalués selon une méthode de filtrage.

7. Procédé selon l'une quelconque des revendications précédentes,
**caractérisé en ce qu'**un signal de comportement normal des organismes d'essai vivants (40a-g) est déterminé dynamiquement par des signaux acoustiques (30a-g) mésurés.

8. Procédé au moins selon la revendication 1,
**caractérisé en ce que** des signaux d'excitation (32a-g) pour la génération des signaux acoustiques Doppler sont irradiés en fonctionnement continu.

9. Procédé au moins selon la revendication 1,
**caractérisé en ce que** des signaux d'excitation (32a-g) pour la génération des signaux acoustiques Doppler sont irradiés en fonctionnement discontinu.

10. Dispositif (10a-g), avec au moins un bac d'essai (12a-g) pour des organismes d'essai vivants (40a-g), au moins une unité capteur acoustique (22a-d, f, g) avec un premier élément capteur acoustique (24a-g), une unité excitateur de son (28a-g), une unité excitateur des signaux (34a-g) configurée à entraîner l'unité excitateur de son (28a-g) et au moins une unité d'évaluation (38a-g) configurée à évaluer les signaux acoustiques (30a-g) mesurés par l'unité capteur acoustique (22ad, f, g),
**caractérisé en ce que** l'élément capteur acoustique (24a-g) comporte un convertisseur piézoélectrique et le dispositif (10a-g) est configuré à exécuter un des procédés selon l'une quelconque des revendications précédentes.

11. Dispositif selon la revendication 10,
**caractérisé en ce que** l'au moins un bac d'essai (12a, c, d, g) pour les organismes d'essai vivants (40a-e) est réalisé par un réservoir d'eau verrouillable.

12. Dispositif selon la revendication 11,
**caractérisé en ce que** le réservoir verrouillable se compose de six éléments de paroi (14c, d, g ; 15c, d, g ; 16c, d, g ; 17c, d, g ; 18c, d, g ; 20c, d, g) et comporte au moins une paroi inclinée (46c-d ; 46g), c'est-à-dire un élément de paroi du réservoir d'eau disposé en un angle, différent d'un angle droit, relative à au moins un autre élément de paroi du réservoir d'eau, pour le bût d'une déflexion des signaux acoustiques (30c-d, 30g).

13. Dispositif selon la revendication 10,
**caractérisé en ce que** l'au moins un bac d'essai (12f) pour les organismes d'essai vivants (40f) est constitué par une cage à grille.

14. Dispositif selon l'une quelconque des revendications 10 à 13,
**caractérisé en ce que** l'au moins une unité capteur acoustique (22b) comporte au moins un premier élément capteur acoustique (24b) pour un enregistrement des signaux acoustiques (30b) le long d'une première direction spatiale de l'au moins un bac d'essai (12b) et comporte au moins un deuxième élément capteur acoustique (26b) pour un enregistrement des signaux acoustiques (30b) le long d'une deuxième direction spatiale de l'au moins un bac d'essai (12b).
